# EUROPÄISCHE PATENTSCHRIFT

(11) **EP 2 173 650 B1**
(45) Veröffentlichungstag und Bekanntmachung des Hinweises auf die Patenterteilung: **15.02.2012**
(21) Anmeldenummer: 08784868.5
(22) Anmeldetag: 18.07.2008
(51) Int. Cl.: B65H 63/032, D04B 35/14, G01N 33/36

(54) **VIELSEITIGE FADENSENSOREINHEIT**
VERSATILE THREAD SENSOR UNIT
UNITÉ DE CAPTEUR DE FILS POLYVALENTE

(30) Priorität: 06.08.2007 DE 102007037004
(43) Veröffentlichungstag der Anmeldung: 14.04.2010
(73) Patentinhaber: Memminger-IRO GmbH, 72280 Dornstetten (DE)
(72) Erfinder: THWAITES, David, Ilkley, West Yorkshire LS 290 PR (GB); KLEINDORP, Makus, 72297 Seewald (DE)
(74) Vertreter: Frese-Göddeke, Beate
(86) Internationale Anmeldenummer: PCT/EP2008/005888
(87) Internationale Veröffentlichungsnummer: WO 2009/018910

(56) Entgegenhaltungen:
- EP-A- 0 519 281
- WO-A-01/07352
- WO-A-2005/066056
- CH-A5- 650 750
- DE-A1- 2 623 856
- DE-A1- 4 105 450
- DE-A1- 4 233 285
- GB-A- 2 358 644

## Beschreibung

Die Erfindung betrifft eine Fadensensoreinheit zur Anwendung an textiltechnischen Einrichtungen, wie beispielsweise Strickmaschinen.

In der Praxis stellt sich immer wieder das Erfordernis zu erfassen, ob ein zu einer Fadenverbrauchsstelle laufender Faden läuft oder steht. Signale für weitere Merkmale liefert ein solcher Sensor aber nicht. Außerdem kann es erforderlich sein, Weiteres herauszufinden, beispielsweise die Fadengeschwindigkeit. Deshalb ist im Zusammenhang mit Fäden für die Textiltechnik eine umfangreiche Sensorik entwickelt worden.

Beispielsweise offenbart die DD 243 518 A1 eine optoelektronische Einrichtung nach dem Reflexlichtschrankenprinzip zur Erkennung von Defekten in textilen Flächengebilden sowie alternativ einen einzelnen Faden. Dazu wird der Sensor als Durchlicht-Lichtschranke ausgebildet.

Die DE 195 25 260 A1 veranschaulicht eine optoelektronische Steuervorrichtung für ein Schussfaden-Messspeichergerät. Der optoelektronische Sensor dient zur Erfassung des Vorbeigangs eines Fadens, der gewissermaßen durch das Sichtfeld des Sensors wischt.

Die Erfassung des Durchgangs eines Fadens durch ein Sensorsichtfeld erzeugt in der Regel ein gut diskriminierbares Signal. Hingegen stellt es sich als schwieriger heraus, das Vorhandensein oder Nichtvorhandensein eines Fadens, der sich durch das Sensorsichtfeld erstreckt, sicher zu erfassen. Dies gilt insbesondere wenn der Sensor ohne besondere Justagemaßnahmen dafür eingerichtet sein soll, mit unterschiedlichsten Fäden zusammen zu arbeiten. Dieses Erfordernis stellt sich, wenn Sensoren für Textilmaschinen bereitgestellt werden sollen, wobei die Sensoren für ein großes spektrum unterschiedlicher Fäden eingerichtet sein sollen.

Aus der DE 26 23 856 A1 ist ein Fadensensor bekannt, der dazu eingerichtet ist, zu erfassen, ob ein laufender oder stehender Faden vorhanden ist oder nicht. Dazu weist der Fadensensor eine Leuchtdiode und einen Fototransistor auf, deren optische Achsen zueinander geneigt sind und mit dem Faden in einer gemeinsamen Ebene liegen. Der Fototransistor ist an eine Auswerteschaltung angeschlossen, um zu erkennen, ob der Fototransistor von dem Faden reflektiertes Licht der Leuchtdiode empfängt oder nicht. Mit diesem Sensor kann aber nicht unterschieden werden, ob der Faden steht oder läuft.

In der WO 2005/066056 A1 ist eine Vorrichtung und ein verfahren zum Erkennen des Vorhandenseins und/oder des Laufens eines Fadens beschrieben.

Die CH 650750 A5 betrifft eine Vorrichtung zur Erfassung eines Garnbruchs bzw. -stops in einer Textilmaschine mit einer Garndurchlasseinrichtung, die einen sich in Garndurchlassrichtung erstreckenden Spalt und Garnführungen aufweist, und mit einer Lichtquelle und einem photoelektrischen Wandler, auf den ein Lichtstrahl der Lichtquelle gerichtet ist. Die Lichtquelle ist an einer Seite der Garndurchlasseinrichtung ortsfest angeordnet und der photoelektrische Wandler an der anderen Seite, wobei die Lichtquelle einen Lichtstrahl konstanter Intensität quer zur Laufrichtung des Fadens aussendet.

Davon ausgehend ist es Aufgabe der Erfindung, einen vielseitig einsetzbaren Fadensensor zu schaffen.

Diese Aufgabe wird mit der Fadensensoreinheit nach Anspruch 1 gelöst:

Die erfindungsgemäße Fadensensoreinheit weist eine Fadenführungseinrichtung auf, mittels derer der Faden durch einen Erfassungsbereich geführt wird und durch die eine Fadenlängsrichtung vorgegeben ist. Sie umfasst einen Fadensensor, der wenigstens einen Sender und wenigstens einen Empfängers aufweist, die in den Erfassungsbereich gerichtet sind. Ein Sender strahlt ein Signal in den Erfassungsbereich ein, das von dem Faden reflektiert werden kann. Der Erfassungsbereich liegt im Sichtfeld eines Empfängers, der das von dem Faden reflektierte Signal erfasst. Ein zu der Fadensensoreinheit gehöriger Signaldiakriminator leitet daraus ein Signal ab, das die Fadensensoreinheit beispielsweise über einen Bus nach außen abgibt oder von ihr abgerufen werden kann. Dieses Signal kann mindestens drei verschiedene Zustände annehmen. Ein erster Signalzustand zeigt an, dass der Faden nicht vorhanden ist, also fehlt. Ein zweiter Signalzustand zeigt an, dass der Faden vorhanden ist, wobei der Faden aber steht (d.h. nicht läuft). Ein dritter Signalzustand zeigt an, dass der Faden vorhanden ist und läuft. Die Signalzustände können auf einer einzigen oder mehreren Leitungen verwirklicht werden. Das Signal kann ein Analogsignal oder ein Digitalsignal sein.

Der Sender und der Empfänger sind in Fadenlängsrichtung von einander beabstandet. Es hat sich herausgestellt, dass diese Anordnung insbesondere zur Erfassung besonders feiner Fäden gut geeignet ist und dass die Fadensensoreinheit sich dadurch durch besonders geringe Störanfälligkeit auszeichnet.

Die Fadensensoreinheit weist einen Halter auf. Der Halter hält lösbar Fadenführungselemente der Fadenführungseinrichtung. Er hält lösbar auch den Fadensensor.

Vorzugsweise sind der Sender und der Empfänger optoelektronische Bauelemente, die mit sichtbarem Licht, infrarotem Licht oder ultravioletem Licht arbeiten. Der Empfänger und der Sender weisen dementsprechend optische Achsen auf, die auch als "Senderichtung" bzw. "Empfangsrichtung" bezeichnet werden. Die Lichtquelle d.h. der Sender kann beispielsweise durch eine Leuchtdiode gebildet sein. Der Empfänger ist beispielsweise ein Fototransistor, ein Fotowiderstand, eine Fotodiode oder ähnliches. Vorzugsweise ist die Lichtquelle eine schmalbandige Lichtquelle. Vorzugsweise ist der Empfänger ebenfalls schmalbandig. Der Sensor und der Empfänger sind hinsichtlich der Wellenlänge vorzugsweise auf einander abgestimmt.

Die optischen Achsen von Sender und Empfänger und die Fadenlängsrichtung liegen vorzugsweise in einer gemeinsamen Ebene. Weiter vorzugsweise schneiden sich die optischen Achsen in einem Punkt auf oder in der Nähe des Fadens. Damit läuft der Faden im Wesentlichen durch das Zentrum des Sendestrahls des Senders sowie durch das Zentrum des Erfassungsbereichs des Empfängers. Dies führt zu einer hohen Signalstärke auch bei dünnen Fäden. Vorzugsweise schließen die optischen Achsen des Senders und Empfängers mit dem Faden einen spitzen Winkel ein. Dies führt bei im Querschnitt kreisförmigem Sendestrahl bzw. kreisförmigem Sichtfeld des Empfängers zu einem elliptischen Erfassungsbereich, wobei die große Längsachse des elliptischen Erfassungsbereichs mit der Fadenlängsrichtung im Wesentlichen übereinstimmt. Somit sind auch sehr dünne und reflexionsschwache Fäden noch in der Lage, ausreichende Signalstärken zu dem Empfänger hin zu reflektieren, so dass das Nutzsignal leicht und sicher von Störsignalen unterschieden werden kann.

Außerdem können viele gleich aufgebaute Fadensensoreinheiten neben einander montiert werden, wobei die Gefahr, dass sich benachbarte Sensoren gegenseitig stören, absolut minimiert ist. Die optischen Achsen benachbarter Fadensensoreinheiten schneiden einander nicht, was gegenseitige Störungen durch Staub oder sonstige Objekte in der Nähe der Fadensensoreinheiten ausschließt. Dies gilt auch dann, wenn der Erfassungsbereich vor der Stirnfläche des Fadensensors im Wesentlichen frei liegt und durch keinerlei Abschirmung eingehaust ist. Dies ergibt bei niedriger oder ganz und gar verschwindender Störempfindlichkeit einen guten manuellen Zugang zu dem Faden und somit eine leichte Handhabung der Fadensensoreinheit.

Die Fadensensoreinheit weist eine.Fadenführungseinrichtung auf, zu der Fadenführungselemente gehören. Diese sind mit einem Halter austauschbar verbunden, um eine Vielfalt von Einsatzmöglichkeiten zu eröffnen. Insbesondere angesichts der von dem Fadensensor erfassbaren großen Vielfalt von verschiedenen Fäden ist es zweckmäßig, wenn verschiedene Fadenführungselement bereitgehalten werden, die für verschiedene Fäden spezifisch eingerichtet sind. Insoweit bilden der Halter, die Sensorelemente und der Fadensensor einen Baukasten der leicht an unterschiedliche Gegebenheiten anpassbar ist.

Weitere Einzelheiten vorteilhafter Ausführungsformen der Erfindung sind Gegenstand der Beschreibung, der Zeichnung oder von Ansprüchen. Die Beschreibung beschränkt sich auf wesentliche Details der Erfindung und sonstiger Gegebenheiten. Die Zeichnung ergänzt die Beschreibung. Es zeigen:
Figur 1 mehrere Fadensensoreinheiten auf einem Maschinenring einer Strickmaschine in perspektivischer Darstellung,
Figur 2 eine Fadensensoreinheit nach Figur 1 mit verschiedenen alternativ vorsehbaren Fadenführungselementen in perspektivischer Explosionsdarstellung,
Figur 3 die Fadensensoreinheit in perspektivischer Darstellung,
Figur 4 die Fadensensoreinheit nach Figur 3 in Explosionsdarstellung,
Figur 5 die Fadensensoreinheit in schematisierter Darstellung zur Veranschaulichung ihrer Geometrie und
Figur 6 die Fadensensoreinheit in schematisierter Perspektivdarstellung zur weiteren Veranschaulichung ihrer Geometrie und ihres Erfassungsbereichs.

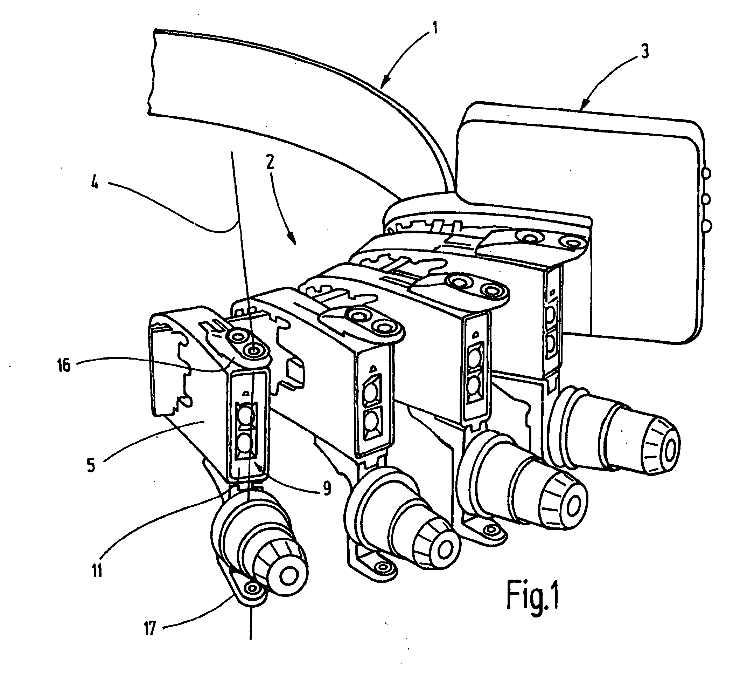

In Figur 1 ist ein beispielsweise zu einer Rundstrickmaschine gehöriger Maschinenring 1 veranschaulicht, auf dem eine Anzahl von untereinander im Wesentlichen gleich ausgebildeten Fadensensoreinheiten 2 sitzen. Diese sind beispielsweise über einen nicht weiter veranschaulichten Bus miteinander sowie mit einer Master-Steuereinheit 3 verbunden, die ebenfalls auf dem Maschinenring 1 sitzen kann. Die Fadensensoreinheiten 2 dienen dazu, zu überwachen, ob ein durchlaufender Faden vorhanden ist oder nicht und ob der vorhandene Faden läuft oder steht. Ein solcher Faden 4 ist in Figur 1 lediglich in Zusammenhang mit einem der Fadensensoreinheiten 2 veranschaulicht.

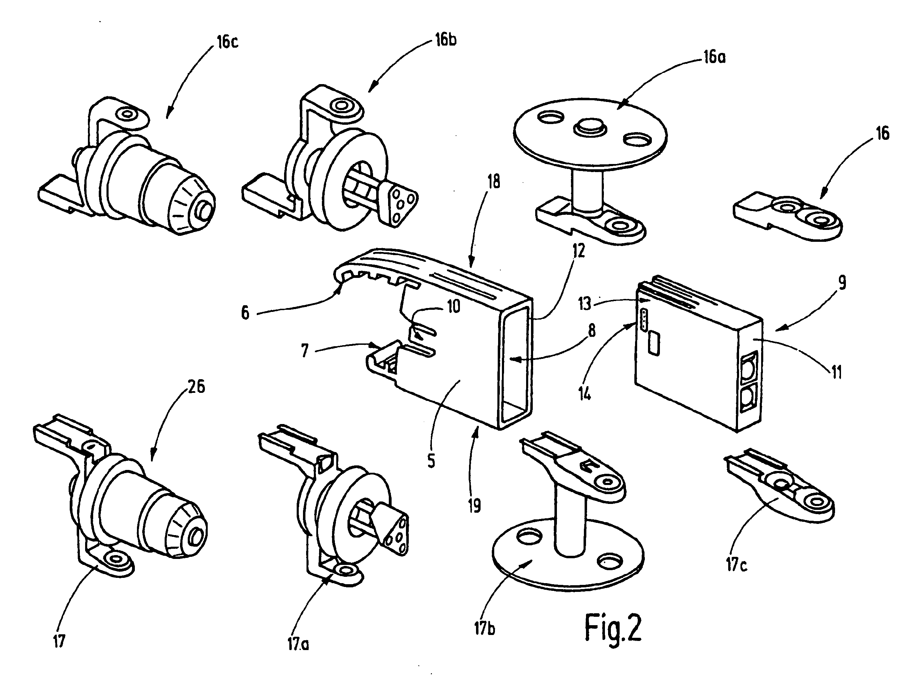

Jede Fadensensoreinheit 2 weist einen Halter 5 auf, der an einem Ende mit dem Maschinenring 1 verrastbar ist. Wie insbesondere aus Figur 2 hervorgeht, kann der Halter 5 dazu mit zwei vorzugsweise etwas federnden Rastfingern 6, 7 versehen sein, die den Maschinenring 1 zwischen einander rastend aufnehmen können.

Der Halter 5 weist in Vorderansicht den Querschnitt eines schmalen aufrecht stehenden Rechtecks auf. Er umschließt einen Innenraum 8 zur Aufnahme eines etwa quaderförmigen Fadensensors 9, der sich in den Innenraum 8 einschieben und dort z.B. mittels einer an dem Halter 5 vorgesehenen Rastzunge 10 rastend sicheren lässt. An seiner vorderseite weist der Fadensensor 9 eine vorzugsweise im Wesentlichen ebene Fläche 11 auf, die mit dem Rand 12 ungefähr abschließt, der die vorderseitige Öffnung des Halters 5 umgibt. Der Querschnitt des Fadensensors 9 stimmt im Wesentlichen mit dem Querschnitt des Innenraums 8 überein. An seiner Rückseite oder, wie in Figur 2 dargestellt, an einer seiner im Wesentlichen flachen Seitenflächen 13 kann der Fadensensor 9 mit einem oder mehreren Steckverbindern 14 versehen sein, um Busleitungen anschließen zu können.

Der Halter 5 und das Gehäuse des Fadensensors 9 sind vorzugsweise aus einem Kunststoff ausgebildet. Dieser kann zur Vermeidung elektrostatischer Aufladungen leitend beschichtet oder insgesamt leitend ausgebildet sein. So können Verschmutzungen des Sensors 9 durch Flusablagerungen vermieden werden.

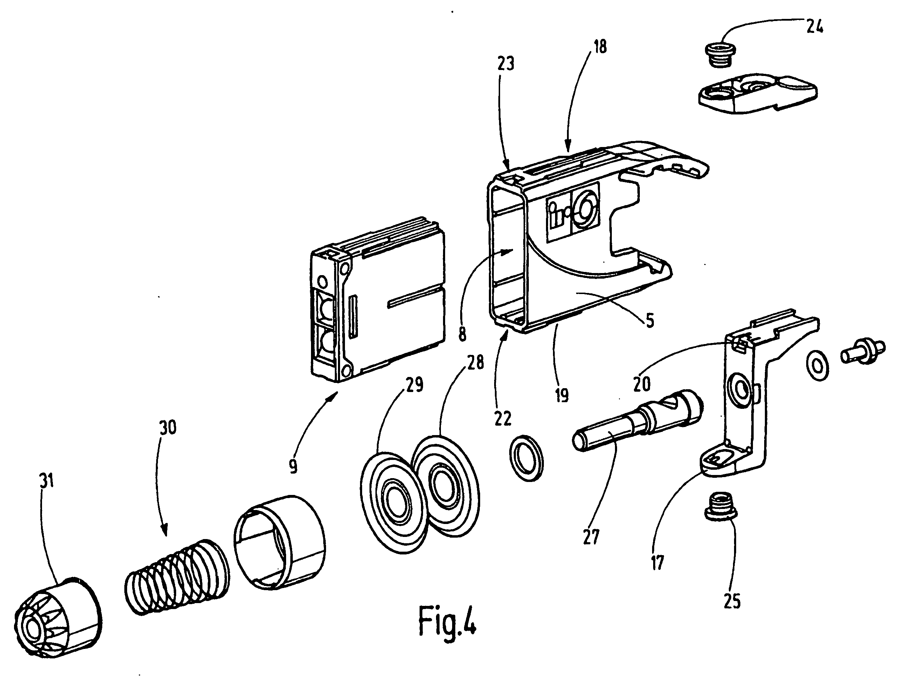

Zu der Fadensensoreinheit 2 gehört eine Fadenführungseinrichtung 15. Diese umfasst zumindest ein, vorzugsweise mehrere Fadenführungselemente 16, 17, die mit dem Halter 5 vorzugsweise lösbar verbunden sind. Der Halter 5 weist dazu, wie aus Figur 2, 3 und 4 hervorgeht, sowohl an seiner schmalen Oberseite wie auch an seiner Unterseite Schiebesitze 18, 19 auf, auf die die Fadenführungselemente 16, 17 aufgeschoben werden können. Die Schiebesitze 18, 19 werden beispielsweise durch an dem Halter 5 ausgebildete Leisten gebildet, die eine Schwalbenschwanzführung bilden. An den Fadenführungselementen 16, 17 sind entsprechende komplementäre Strukturen ausgebildet. Außerdem sind an den Fadenführungselementen 16, 17 vorzugsweise entsprechende Rastmittel, beispielsweise in Form von Rastzungen 20 vorgesehen, wie sie anhand des Fadenführungselements 17 in Figur 4 veranschaulicht sind. Solchen Rastzungen sind dann an den Schiebesitzen 18, 19 vorgesehene Rastausnehmungen 22, 23 zugeordnet.

Auch die Fadenführungselemente 16, 17 sind vorzugsweise aus einem Kunststoff ausgebildet, der zur Vermeidung elektrostatischer Aufladungen leitend beschichtet oder insgesamt leitend ausgebildet oder mit einem elektrischen Leiter versehen sein kann. So können insbesondere die mit dem Faden 4 in Verbindung stehenden Teile geerdet werden, um Verschmutzungen des Sensors 9 durch Flusablagerungen zu vermeiden.

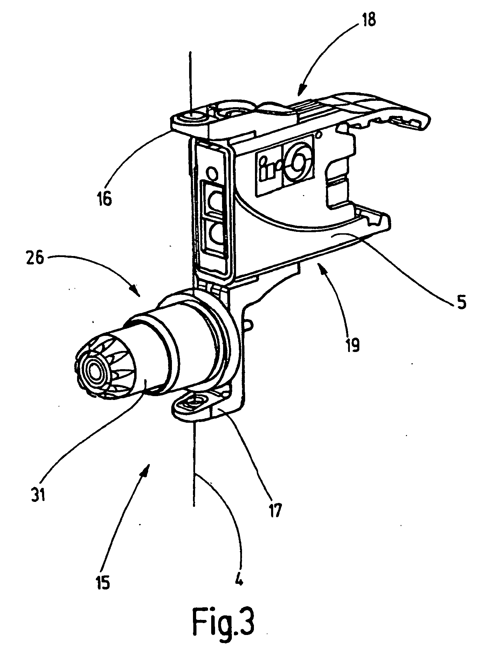

Die Fadenführungselemente 16, 17 enthalten beispielsweise Fadenführungsösen 24, 25, z.B. als Keramikeinsätze und legen einen, wie Figur 3 veranschaulicht, in Gebrauch vertikalen Fadenlaufweg fest. Außerdem kann an einem der Fadenführungselemente, beispielsweise dem unteren Fadenführungselement 17, ein weiteres Organ, wie beispielsweise eine Fadenbremse 26 angeordnet sein. Figur 4 veranschaulicht eine Ausführungsform mit zentralem Lagerstift 27, Bremstellern 28, 29, einer Vorspannfeder 30, einer Spannmutter 31 sowie weiteren Kleinteilen.

Dem Halter 5 können, wie insbesondere Figur 2 veranschaulicht, weitere Fadenführungselemente 16a, 16b und 16c sowie 17a, 17b und 17c in unterschiedlichen Ausführungsformen zugeordnet sein. Die oben und unten an dem Halter 5 zu befestigenden Fadenführungselemente können untereinander gleich ausgebildet sein. Beispielsweise kann das Fadenführungselement 17c dem Fadenführungselement 16 und das Fadenführungselement 16c dem Fadenführungselement 17 entsprechen. Weitere Fadenführungselemente 16a, 17b können beispielsweise als Umschlingungsbremsen ausgebildet sein. Die Fadenbremse kann, wie die Fadenführungselemente 16b, 17a zeigen, auch als federlose Bremse mit magnetisch gegeneinander gespannten Bremstellern ausgebildet sein. Auf diese Weise kann der Halter 5 mit verschiedenen Fadenführungselementen 16 bis 16c und 17 bis 17c einen Baukasten bilden, mit dem sich die Fadensensoreinheit an die verschiedensten Garnqualitäten anpassen lässt. Außerdem können von dem Innenraum 8 die unterschiedlichsten Fadensensoren 9 aufgenommen werden. Im Einzelfall kann der Innenraum auch unbesetzt bleiben, wenn beispielsweise einer der vielen zu der Strickmaschine gelieferten Fäden nicht überwacht werden muss.

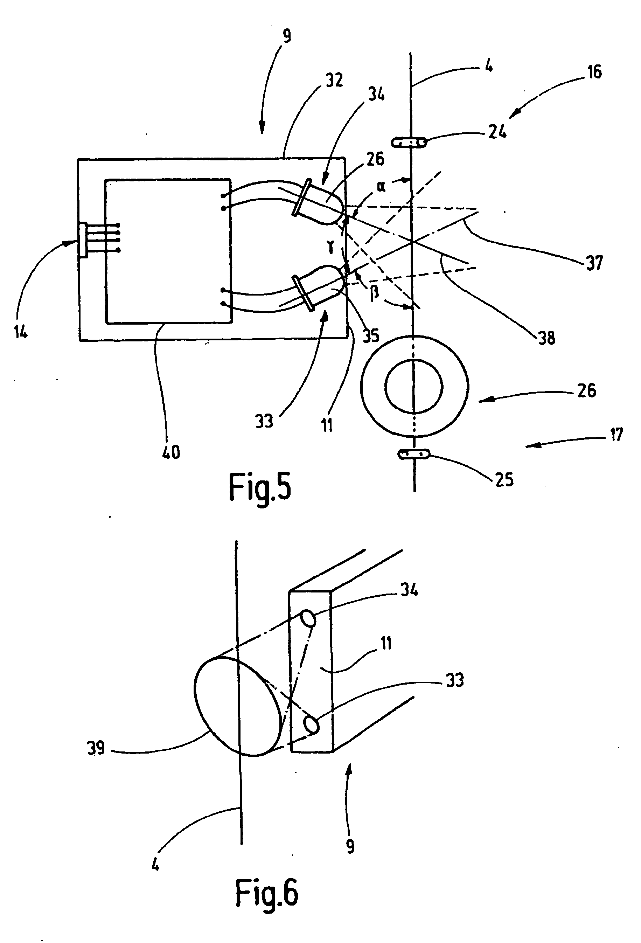

Figur 5 veranschaulicht die Ausbildung des Fadensensors 9 schematisch. In seinem Gehäuse 32 sind zumindest ein Sender 33 und ein Empfänger 34 angeordnet. Der Sender 33 ist beispielsweise eine Leuchtdiode 35 oder eine sonstige Lichtquelle. Der Empfänger 34 ist beispielsweise ein Fototransistor 36 oder ein sonstiges lichtempfindliches elektrisches Bauelement. Der Fadensensor 9 ist für den Betrieb mit unterschiedlichen Betriebsspannungen eingerichtet, die z.B. in dem Bereich von 12 V bis 24 V liegen. Der Fadensensor 9 ist dazu eingerichtet, folgende Zustände zu unterscheiden:
- Faden nicht vorhanden,
- Faden vorhanden und Faden läuft nicht,
- Faden vorhanden und läuft.

Dazu sendet der Sender 33 gepulstes Licht zu dem Faden 4. Die Pulsdauer beträgt z.B. 30 µs, während der Pulsabstand z.B. 2,5 ms beträgt. Die Speisung der LED, die den Sender 33 bildet, erfolgt vorzugsweise über einen Kondensator, der so ausgelegt ist, dass er die LED für die vorgesehene Pulsdauer speist. Die Lichtintensität nimmt während des Lichtpulses ab. Der Empfänger 34 ist ständig aktiv, wird aber vorzugsweise nur während der Dauer des Lichtimpulses vorzugsweise mehrfach abgefragt. Gegebenenfalls kann der Empfänger zusätzlich ein- oder mehrfach während der Pause zwischen zwei Lichtpulsen abgefragt werden. Der Fadensensor 9 wertet die Differenz des empfangenen Lichts zwischen Empfang in der Pulspause und Empfang während des Lichtpulses aus. Dadurch wird das Vorhandensein bzw. Nichtvorhandensein des Fadens 4 erfasst. Aus Veränderungen des während des Sendebetriebs des Senders 3 empfangenen Lichts kann unterschieden werden, ob der Faden 4 steht oder läuft. Hierzu wird z.B. das Spektrum des empfangenen Signals ausgewertet. Oder es wird eine Rauschanalyse durchgeführt. Enthält das empfangene Signal Rauschanteile oberhalb einer Grenzfrequenz, wird darauf geschlossen, dass der Faden läuft. Fehlen solche Rauschanteile wird darauf geschlossen, dass der Faden steht.

Die von dem Fadensensor 9 erfassten drei unterschiedlichen Zustände des Fadens (fehlt, steht, läuft) können in einem in dem Fadensensor vorhandenen extern abfragbaren Speicher abgespeichert werden. Dies kann, falls erforderlich, auch im Zusammenhang mit einer Bezugsgröße, wie z.B. Zeit, Maschinentakt, Maschinenwinkel usw. geschehen.

Der Sender 33 und der Empfänger 34 sind vorzugsweise übereinander angeordnet. Bei der dargestellten Ausführungsform verläuft der Faden 4 vertikal, wobei der Sender 33 unten und der Empfänger 34 oben angeordnet sind. Der Sender 33 legt einen Öffnungswinkel fest und sendet Licht beispielsweise kegelförmig bezüglich einer mittig in dem Kegel liegenden optischen Achse 37 aus. Diese optische Achse 37 markiert die Senderichtung des Senders 33. Der Empfänger 34 hat einen ebenfalls kegelförmigen, zu einer optischen Achse 38 konzentrischen Erfassungsbereich. Die optische Achse 38 bezeichnet die Empfangsrichtung. Während die optische Achse 37 des Senders 33 vorzugsweise schräg nach oben gerichtet ist, ist die optische Achse 38 des Empfängers vorzugsweise nach unten geneigt gerichtet. Diese Anordnung führt zu einer geringen Störempfindlichkeit im Hinblick auf Fremdlicht, das z.B. von einer Raumbeleuchtung herrühren kann. Der Sensor "schaut" schräg nach unten und nimmt Störlichtquellen weniger oder nicht wahr. Außerdem lagert sich weniger Flus auf seinem Sichtfenster ab, wenn dieses vertikal oder schräg nach unten geneigt angeordnet ist. Flusablagerungen auf dem Fenster des Senders stören hingegen weniger.

Die genannte Anordnung ist aber nicht zwingend. Es können auch der Sender 33 oben und der Empfänger 34 unten angeordnet werden.

Die optischen Achsen 37, 38 sind vorzugsweise in einer gemeinsamen vertikal orientierten Ebene angeordnet, innerhalb derer auch der Faden 4 verläuft. Dies gilt zumindest im Rahmen der Genauigkeit der durch die Fadenführungselemente 16, 17 vorgegebenen Fadenführung. Haben beispielsweise die Fadenführungsösen 24, 25 jeweils einen Durchmesser von einigen Millimetern, kreuzen sich die optischen Achsen 37, 38 und der Faden 4 innerhalb eines Volumens mit einem Durchmesser von ebenfalls mehreren Millimetern. Sie müssen sich demgemäß nicht zwangsläufig in einem mathematischen Punkt schneiden.

Vorzugsweise schließen der Faden 4 und die optische Achse 38 miteinander einen spitzen Winkel a ein. Ebenso schließen die optische Achse 37 und der Faden 4 miteinander vorzugsweise einen spitzen Winkel β ein. Des Weiteren schließen die optischen Achsen 37, 38 miteinander vorzugsweise einen spitzen Winkel γ ein. Letzterer Winkel γ kann jedoch auch ein rechter Winkel oder im Extremfall ein stumpfer Winkel sein.

Die Anordnung des Senders 33 und Empfängers 34 in spitzen Winkeln zu dem Faden 4 führt gemäß Figur 6 dazu, dass der Faden 4 den Sendekegel des Senders 33 und den Empfangskegel des Empfängers 34 jeweils schräg d.h. in einer zu der Stirnfläche 11 parallelen Ebene schneidet, in der der entsprechende Kegelschnitt eine Ellipse 39 ist. Diese Ellipse 39 markiert den Erfassungsbereich des Fadensensors 9. Der Faden 4 verläuft im Wesentlichen entlang ihrer großen Längsachse. Somit wird von dem Sender 33 ein relativ langer Abschnitt des Fadens 4 bestrahlt. Der Empfänger 34 erfasst ebenfalls einen relativ langen Abschnitt des Fadens 4. So kann auch ein sehr dünner und reflexionsarmer Faden ein ausreichendes Reflexsignal liefern.

Zugleich ist die Gefahr vermindert, dass der Empfänger 34 Signale erhält, die von dem Sender eines benachbarten Sensors herrühren. Dies vor allem wegen der Ausrichtung der optischen Achsen 37, 38 in einer Vertikalebene, die bezogen auf den Maschinenring nach Figur 1 radial zu dieser orientiert ist. Somit sind die Sender und Empfänger jeder der Fadensensoreinheiten 2 in einer jeweils eigenen Radialebene ausgerichtet.

Der Fadensensor 9 enthält eine interne Schaltung 40, an die der Sender 33 und der Empfänger 34 angeschlossen sind. Vorzugsweise wird der Sender 33 gepulst betrieben. Ebenso kann der Empfänger 34 im Takte des gepulsten Signals abgefragt werden. Alternativ kann er an ein Filter angeschlossen sein, das lediglich Signale mit der Pulsfrequenz des Senders 33 hindurch dringen lässt. Damit lassen sich Störsignale ausschließen. Es kann zur Vermeidung von Beeinflussungen benachbarter Sensoreinheiten 2 des Weiteren hilfreich sein, wenn verschiedene Sensoreinheiten 2 mit unterschiedlichen Pulsfrequenzen arbeiten.

Die insoweit beschriebene Fadensensoreinheit 2 arbeitet wie folgt:

Sie dient der Erkennung des Fadens 4 und der Anzeige, ob dieser vorhanden ist oder nicht. Wenn der Faden 4 durch die Fadenführungselemente 16, 17 verlaufend angeordnet ist, befindet er sich in dem Erfassungsbereich 39. Von dem Sender 33 abgegebenes infrarotes sichtbares oder ultraviolettes dauernd anliegendes oder gepulstes Licht trifft den Faden 4. Es wird teilweise reflektiert und von dem Empfänger 34 empfangen. Die Schaltung 40 erkennt dies und schließt daraus auf das Vorhandensein des Fadens 4.

Ist der Faden 4 gerissen oder fehlt er, empfängt der Empfänger 34 kein von dem Faden 4 reflektiertes Licht. Die Schaltung 40 registriert dies und gibt demzufolge ein Signal an einen über den Steckverbinder 14 angeschlossenen Bus oder sonstige Leitungen ab, um dies an die Master-Steuereinheit 3 zu melden.

Die Fadensensoreinheit 2 kann des Weiteren dazu eingerichtet sein, andere Kenngrößen, beispielsweise die Fadengeschwindigkeiten zu erfassen. Dies kann beispielsweise anhand des von dem Empfänger 34 empfangenen Signals erfolgen, indem dessen Spektrum ausgewertet wird. Je schneller der Faden läuft, desto akzentuierter treten hochfrequente Anteile des Spektrums des Empfangssignals auf.

Des Weiteren ist es möglich, über einen beispielsweise über den Steckverbinder 14 angeschlossenen Bus eine Aktivierung oder Deaktivierung der Fadensensoreinheit 2 vorzunehmen.

Ist ein Faden gerissen, kann er wegen der freien Zugänglichkeit der Fadenführungselemente 16, 17 leicht neu eingefädelt werden. Der Erfassungsbereich 39 liegt vollkommen frei vor der Stirnfläche 11 des Fadensensors 9. In diesem Bereich können somit auch keine Flusablagerungen entstehen, die fehlerhafte Lichtreflexe und somit ein fälschliches Vorhandensein des Fadens signalisieren könnten, wenn dieser tatsächlich nicht vorhanden ist.

Eine erfindungsgemäße Fadensensoreinheit 2 ist als Baukastensystem ausgebildet und enthält neben einem Halter 5, der wahlweise mit verschiedensten Fadenführungselementen 16 bis 16c, 17 bis 17c zu verbinden ist, ein Aufnahmefach für einen Fadensensor 9. Dieser weist in Fadenlängsrichtung hinter einander angeordnete Elemente zum Senden und Empfangen elektromagnetischer Strahlung (Licht) auf. Die Elemente weisen zueinander geneigte optische Achsen 37, 38 auf, die mit dem Faden 4 vorzugsweise in einer gemeinsamen, vorzugsweise vertikalen Ebene liegen.

### Bezugszeichen:

- 1: Maschinenring
- 2: Fadensensoreinheit
- 3: Mastersteuereinheit
- 4: Faden
- 5: Halter
- 6, 7: Rastfinger
- 8: Innenraum
- 9: Fadensensor
- 10: Rastzunge
- 11: Stirnfläche
- 12: Rand
- 13: Flachseite
- 14: Steckverbinder
- 15: Fadenführungseinrichtung
- 16, 17: Fadenführungselemente
- 18, 19: Schiebesitze
- 20: Rastzunge
- 22, 23: Rastausnehmungen
- 24, 25: Fadenführungsösen
- 26: Fadenbremse
- 27: Lagerstift
- 28, 29: Bremsteller
- 30: Vorspannfeder
- 31: Spannmutter
- 32: Gehäuse
- 33: Sender
- 34: Empfänger
- 35: Leuchtdiode
- 36: Fototransistor
- 37, 38: optische Achsen
- 39: Erfassungsbereich
- 40: Schaltung

## Patentansprüche

1. Fadensensorelnhelt (2)
mit einer Fadenführungseinrichtung (15) zur Führung eines Fadens (4) durch einen Erfassungsbereich (39), durch die eine Fadenlängsrichtung vorgegeben ist,
mit einem Fadensensor (9), der wenigstens einen Sender (33) und wenigstens einen Empfänger (34) aufweist, die in den Erfassungsbereich (39) gerichtet sind, und
mit einem Halter (5), der Fadenführungselemente (16, 17) der Fadenführungseinrichtung (15) lösbar hält,
**dadurch gekennzeichnet, dass** der Sender (33) und der Empfänger (34) in der Fadenlängsrichtung voneinander beabstandet sind,
dass der Fadensensor (9) darauf eingerichtet ist, folgende Zustände zu unterscheiden:
- Faden nicht vorhanden,
- Faden vorhanden und Faden läuft nicht,
- Faden vorhanden und läuft, und
dass der Halter (5) den Fadensensor (9) lösbar hält.

2. Fadensensoreinheit nach Anspruch 1, **dadurch gekennzeichnet, dass** der Sender (33) eine Lichtquelle (35) und der Empfänger (34) ein lichtempfindliches Bauelement (36) ist.

3. Fadensensoreinheit nach Anspruch 1, **dadurch gekennzeichnet, dass** der Sender (33) und der Empfänger (34) jeweils eine Senderichtung (37) bzw. eine Empfangsrichtung (38) festlegen und dass die Sendrichtung (37) und die Empfangsrichtung (38) mit der Fadenlängsrichtung in einer gemeinsamen Ebene liegen.

4. Fadensensoreinheit nach Anspruch 1, **dadurch gekennzeichnet, dass** der Sender (33) und der Empfänger (34) jeweils eine Senderichtung (37) bzw. eine Empfangsrichtung (38) festlegen und dass die Senderichtung (37) und die Empfangsrichtung (38) mit der Fadenlängsrichtung jeweils einen spitzen Winkel (α, β) einschließen.

5. Fadensensoreinheit nach Anspruch 1, **dadurch gekennzeichnet, dass** der Sender (33) und der Empfänger (34) jeweils eine Senderichtung (37) bzw. eine Empfangsrichtung (38) festlegen, die sich mit dem Faden (4) in einem gemeinsamen Punkt treffen.

6. Fadensensoreinheit nach Anspruch 1, **dadurch gekennzeichnet, dass** der Fadensensor (9) von einem Aufnahmefach (8) des Halters (5) vollständig aufgenommen ist.

7. Fadensensoreinheit nach Anspruch 1, **dadurch gekennzeichnet, dass** der Fadensensor (9) an seiner dem Erfassungsbereich (39) zugewandten Seite eine flache Stirnfläche (11) aufweist.

8. Fadensensoreinheit nach Anspruch 1, **dadurch gekennzeichnet, dass** der Fadensensor (9) ein elektrisch leitfähiges Gehäuse aufweist.

9. Fadensensoreinheit nach Anspruch 6, **dadurch gekennzeichnet, dass** der Halter (5) elektrisch leitfähig ausgestaltet ist und/oder
an dem Halter (5) Fadenleitelemente (24, 25) vorgesehen sind, die elektrisch geerdet sind.

10. Fadensensoreinheit nach Anspruch 1, **dadurch gekennzeichnet, dass** der Sender (33) gepulstes Licht zu dem Faden (4) aussendet.

11. Fadensensoreinheit nach Anspruch 10, **dadurch gekennzeichnet, dass** die Speisung des Senders (33) über einen Kondensator erfolgt, der so ausgelegt ist, dass er den Sender (33) für die vorgesehene Pulsdauer speist.

12. Fadensensoreinheit nach Anspruch 10, **dadurch gekennzeichnet, dass** die Sendeintensität des Senders (33) während des Lichtpulses abnimmt.

13. Fadensensoreinheit nach Anspruch 12. **dadurch gekennzeichnet, dass** der Fadensensor (9) die Differenz des empfangenen Signals zwischen Empfang in der Pulspause und Empfang während des Sendepulses auswertet, um das Vorhandensein bzw. Nichtvorhandensein des Fadens (4) zu erfassen.

14. Fadensensoreinheit nach Anspruch 1, **dadurch gekennzeichnet, dass** der Fadensensor (9) aus Veränderungen des während des Sendebetriebs des Senders (3) empfangenen Signals unterscheidet, ob der Faden (4) steht oder läuft.

15. Fadensensoreinheit nach Anspruch 1, **dadurch gekennzeichnet, dass** die von dem Fadensensor (9) erfassten unterschiedlichen Zustände des Fadens (4) in einem in dem Fadensensor (9) vorhandenen Speicher abgespeichert werden.

## Claims

1. Thread sensor unit (2)
with a thread guiding device (15) for guiding a thread (4) through a detecting area (39), by which a thread longitudinal direction is predetermined,
with a thread sensor (9), which comprises at least one transmitter (33) and at least one receiver (34), which are directed into the detecting area (39) and
with a holder (5) which holds the thread guiding elements (16, 17) of the thread guiding device (15) in a releasable manner, **characterised in that** the transmitter (33) and the receiver (34) are spaced apart from one another in the thread longitudinal direction, **in that** the thread sensor (9) is set up to distinguish the following states:
- thread not present,
- thread present and thread not running
- thread present and running, and
**in that** the holder (5) holds the thread sensor (9) in a releasable manner.

2. Thread sensor unit according to claim 1, **characterised in that** the transmitter (33) is a light source (35) and the receiver (34) a light-sensitive component (36).

3. Thread sensor unit according to claim 1, **characterised in that** the transmitter (33) and the receiver (34) respectively determine a transmission direction (37) and a receiving direction (38) and **in that** the transmission direction (37) and the receiving direction (38) lie in the same plane as the thread longitudinal direction.

4. Thread sensor unit according to claim 1, **characterised in that** the transmitter (33) and the receiver (34) respectively determine a transmission direction (37) and a receiving direction (38) and **in that** the transmission direction (37) and the receiving direction (38) enclose respectively an acute angle (α, β) with the thread longitudinal direction.

5. Thread sensor unit according to claim 1, **characterised in that** the transmitter (33) and the receiver (34) respectively determine a transmission direction (37) and a receiving direction (38) which meet with the thread (4) at a common point.

6. Thread sensor unit according to claim 1, **characterised in that** the thread sensor (9) is mounted completely by a holding compartment (8) of the holder (5).

7. Thread sensor unit according to claim 1, **characterised in that** the thread sensor (9) has a flat end face (11) on its side facing the detecting area (39).

8. Thread sensor unit according to claim 1, **characterised in that** the thread sensor (9) comprises an electrically conductive housing.

9. Thread senor unit according to claim 6, **characterised in that** the holder (5) is designed to be electrically conductive and/or on the holder (5) thread guiding elements (24, 25) are provided which are electrically earthed.

10. Thread senor unit according to claim 1, **characterised in that** the transmitter (33) transmits pulsed light to the thread (4).

11. Thread sensor unit according to claim 10, **characterised in that** the transmitter (33) is supplied via a capacitor, which is designed so that it supplies the transmitter (33) for the intended pulse duration.

12. Thread sensor unit according to claim 10, **characterised in that** the transmission intensity of the transmitter (33) is reduced during the light pulse.

13. Thread sensor unit according to claim 12, **characterised in that** the thread sensor (9) evaluates the difference of the received signal between receiving in the pulse interval and receiving during the transmission pulse, in order to determine the presence or absence of the thread (4).

14. Thread sensor unit according claim 1, **characterised in that** the thread sensor (9) distinguishes from changes in the signal received during the transmission operation of the transmitter (3) whether the thread (4) is not running or is running.

15. Thread sensor unit according to claim 1, **characterised in that** the different states of the thread (4) determined by the thread sensor (9) are stored in a memory provided in the thread sensor (9).

## Revendications

1. Unité de capteur de fils (2) comprenant
un dispositif de guidage de fils (15) pour le guidage d'un fil (4) par une zone de détection (39), par laquelle un sens longitudinal de fil est prescrit,
un capteur de fils (9) qui présente au moins un émetteur (33) et au moins un récepteur(34) qui sont dirigés dans la zone de détection (39), et
un support (5) qui maintient de manière détachable des éléments de guidage de fils (16, 17) du dispositif de guidage de fils (15),
**caractérisée en ce que** l'émetteur (33) et le récepteur (34) sont espacés l'un de l'autre dans le sens longitudinal de fil,
**en ce que** le capteur de fils (9) est aménagé afin de différencier les états suivants :
absente de fil,
- présence de fil et non circulation de fil,
- présence de fil et circulation de fil, et
**en ce que** le support (5) maintient de manière détachable le capteur de fil (9).

2. Unité de capteur de fils selon la revendication 1, **caractérisée en ce que** l'émetteur (33) est une source de lumière (35) et le récepteur (34) est un élément de construction (36) photosensible.

3. Unité de capteur de fils selon la revendication 1, **caractérisée en ce que** l'émetteur (33) et le récepteur (34) définissent respectivement un sens d'émission (37) ou un sens de réception (38) et **en ce que** le sens d'émission (37) et le sens de réception (38) se trouvent dans un plan commun avec le sens longitudinal de fil.

4. Unité de capteur de fils selon la revendication 1, **caractérisée en ce que** l'émetteur (33) et le récepteur (34) définissent respectivement un sens d'émission (37) ou un sens de réception (38) et **en ce que** le sens d'émission (37) et le sens de réception (38) forment avec le sens longitudinal de fil respectivement un angle aigu (α, β).

5. Unité de capteur de fils selon la revendication 1, **caractérisée en ce que** l'émetteur (33) et le récepteur (34) définissent respectivement un sens d'émission (37) ou un sens de réception (38) qui se rencontrent avec le fil (4) en un point commun.

6. Unité de capteur de fils selon la revendication 1, **caractérisée en ce que** le capteur de fils (9) est entièrement logé dans une case de réception (8) du support (5).

7. Unité de capteur de fils selon la revendication 1, **caractérisée en ce que** le capteur de fils (9) présente une surface frontale plate (11) sur son côté tourné vers la zone de détection (39).

8. Unité de capteur de fils selon la revendication 1, **caractérisée en ce que** le capteur de fils (9) présente un boîtier électroconducteur.

9. Unité de capteur de fils selon la revendication 6, **caractérisée en ce que** le support (5) est équipé de manière électroconductrice et/ou des éléments guide-fils (24, 25) mis à la terre sont prévus sur le support (5).

10. Unité de capteur de fils selon la revendication 1, **caractérisée en ce que** l'émetteur (33) émet de la lumière pulsée vers le fil (4).

11. Unité de capteur de fils selon la revendication 10, **caractérisée en ce que** l'alimentation de l'émetteur (33) est effectuée par un condensateur qui est configuré de sorte à alimenter l'émetteur (33) pour la durée de pulsation prévue.

12. Unité de capteur de fils selon la revendication 10, **caractérisée en ce que** l'intensité d'émission de l'émetteur (33) décroît pendant l'impulsion lumineuse.

13. Unité de capteur de fils selon la revendication 12, **caractérisée en ce que** le capteur de fil (9) évalue la différence de signal reçu entre la réception dans l'intervalle d'impulsion et la réception pendant l'impulsion d'émission afin de détecter la présence ou l'absence du fil (4).

14. Unité de capteur de fils selon la revendication 1, **caractérisée en ce que** le capteur de fils (9) différencie si le fil (4) s'arrête ou circule à partir de modifications du signal reçu pendant le mode d'émission de l'émetteur (3).

15. Unité de capteur de fils selon la revendication 1, **caractérisée en ce que** les différents états détectés par le capteur de fils (9) du fil (4) sont enregistrés dans une mémoire présente dans le capteur de fils (9).
